# EUROPEAN PATENT SPECIFICATION

(11) **EP 2 097 056 B1**
(45) Date of publication and mention of the grant of the patent: **24.11.2010**
(21) Application number: 07849439.0
(22) Date of filing: 12.12.2007
(51) Int. Cl.: A61H 39/02, A61N 1/08, A61B 5/04, A61N 1/32

(54) **ELECTROTHERAPEUTIC DEVICE**
ELEKTROTHERAPEUTISCHES GERÄT
DISPOSITIF ÉLECTROTHÉRAPEUTIQUE

(30) Priority: 18.12.2006 EP 06126316
(43) Date of publication of application: 09.09.2009
(73) Proprietor: Koninklijke Philips Electronics N.V., 5621 BA Eindhoven (NL)
(72) Inventor: VAN HERK, Johannes J., 5656 AE Eindhoven (NL); VAN LIESHOUT, Marjolein I., 5656 AE Eindhoven (NL); WAGEMAKERS, Femke, 5656 AE Eindhoven (NL); KOLEN, Alexander F., 5656 AE Eindhoven (NL); BODLAENDER, Maarten P., 5656AE Eindhoven (NL); DROST, Mirelva M., 5656AE Eindhoven (NL)
(74) Representative: Uittenbogaard, Frank
(86) International application number: PCT/IB2007/055041
(87) International publication number: WO 2008/075250

(56) References cited:
- EP-A- 0 784 960
- WO-A-98/46129
- US-A- 5 505 208
- US-B1- 6 301 500
- KWOK G ET AL: "MAPPING ACUPUNCTURE POINTS USING MULTI CHANNEL DEVICE" AUSTRALASIAN PHYSICAL AND ENGINEERING SCIENCES IN MEDICINE, ASTRALASIAN COLLEGE OF PHYSICAL SCIENTISTS IN, AU, vol. 21, no. 2, 1998, pages 68-72, XP000911092 ISSN: 0158-9938

## Description

### FIELD OF THE INVENTION

The present invention generally relates to electro-therapeutic devices. In particular, the present invention relates to an electrotherapeutic device, comprising a plurality of electrodes, measuring means operably electrically connected to the plurality of electrodes and arranged to measure a value of an electrical quantity for each of a plurality of said electrodes, and a control unit coupled to the measuring means and arranged to process the measured values.

The device can be used as a professional or consumer product for pain relief in the medical and paramedical field.

### BACKGROUND OF THE INVENTION

Document US 6,301,500 discloses an electro-stimulation apparatus using an electrode matrix with a counterelectrode. The electrodes can measure local electrical impedance, and may also be used to provide electro-stimulation.

However, the known device is not always suitable for use by non-professional users. It still requires knowledge to determine whether the electrode matrix is properly positioned, since the user needs to know whether a position having a certain electrical impedance corresponds to a treatment for one condition or to that of another. In general, a disadvantage of the known device is that is not able to determine or check a correct position for treatment.

Document EP 0 784 960 discloses an electrocardiographic measurement which may be performed by a nonexpert via attaching an electrocardiographic electrode assembly to a spot on the body that is roughly inside the region where the electrocardiogram can be expected to appear. An optimum signal discriminating circuit selects, from a plurality of electrodes, that one which is most suitable for a measurement. The electrocardiograph receives the selected ECG signals, stores them in a memory, and transmits an electrocardiographic pattern to a doctor for diagnosis. However, the electrocardiograph only searches for the electrodes where the strongest signal comes from and selects this electrode as measuring electrode. As such, any misalignment of the plurality of electrodes may be compensated for. By using the disclosure of EP 0 784 960 it is not possible to determine where on the body the plurality of electrodes are attached and whether the recorded signal actually comes from the sensing location which was targeted.

Document WO 98/46129 discloses a generally flat, rectangular rubber pad support discrete, non-invasive electrodes in a 7 by 9 array and a ground electrode to collect surface electromyographic (EMG) signals from an underlying muscle group of a patient. The electronic apparatus conditions the EMG signals and is programmed to survey the electrode array simultaneously to provide a description of the activity of individual muscles in the muscle group at that point in time. The description is displayed on a video monitor or the like, juxtaposed over a display of normal muscle anatomy, with differences being emphasized by color enhancement. The electrode pad is particularly suited for the lower back muscle groups and may be retained in position by a lumbar support belt having a pad molded to the contours of the human spine. The position of the array of electrodes must be relatively accurately known to be able to distinguish between normal muscle anatomy and the viewed muscle anatomy. For that reason, the location of the array of electrodes is aligned to the skeletal structure of the patient. This alignment requires much physiological skills. Furthermore, no pattern recognition is introduced for determining the location of the array of electrodes on the human or animal body.

### OBJECT OF THE INVENTION

It is an object of the present invention to provide a device of the kind mentioned above that is able to determine or check a correct position of the device on a body part to be treated.

### SUMMARY OF THE INVENTION

The object of the invention is achieved with an electro-therapeutic device for application on a human or animal body part, according to claim 1, comprising a plurality of electrodes, measuring means operably electrically connected to the plurality of electrodes and arranged to measure a value of an electrical quantity for each electrode in at least a subset of the plurality of electrodes and a control unit coupled to the measuring means and arranged to process the measured values, wherein the control unit further comprises pattern recognition means arranged to determine an electrode position on the human or animal body using pattern recognition on a pattern in the measured values.

The device according to the present invention is able to determine patterns in the measured electrical quantity, in dependence of electrode position. This enables the device to carry out a check on the position of the device on the body part to be treated, or even determine a correct positioning by itself. Thereto, use is made of the insight that for each treatment and each body part, certain points on the body should be treated, which is per se comparable to treatment points in e.g. acupuncture. It turns out that such points always show a certain position pattern, that may be compared to constellations in the sky and that have a certain relative position on the body. By recognizing such constellations, the device is thus able to 'navigate' across the body part, and determine whether or not a correct position of the electrodes has been given. The device could even determine by itself where the desired positions for treatment are, as will be shown below. Importantly, user's knowledge of such points is no longer necessary, and the device is more user friendly.

The measuring means are arranged to measure a value of an electrical quantity for each electrode in at least a subset of the plurality of electrodes. This subset may comprise the full plurality of electrodes, but could also comprise a part thereof, for example if only a part of the electrodes is actually applied.

Particular and preferred embodiments will be disclosed in the dependent claims and described in the following.

In particular, the electrical quantity comprises skin impedance, skin resistance or a relative sensory perception. These quantities are meant to comprise also current, preferably at a fixed voltage, and voltage, preferably at a fixed current, which are known to also provide impedance or resistance. It is furthermore possible to determine the quantities at ac, at any desired and suitable frequency, or dc. The relative sensory perception is also useful, as it is also an indication of physiologically active points. The relative skin sensory perception could e.g. relate to pain, temperature, a tingling sensation, et cetera, although these are somewhat more difficult to measure. Any other suitable electrical quantity could also be measured, such as a capacitive value.

In an embodiment, the pattern comprises a subgroup of the plurality of electrodes that indicate a local extreme value of said electrical quantity, preferably positions of the electrodes of the subgroup on said body part. In other words, the device determines which electrodes indicate, i.e. are positioned on or directly adjacent, a spot of a local extreme value of the quantity, and preferably a position of those electrodes. On the basis of the positions thus determined, appropriate action may be taken.

Extreme values may be determined locally, as mentioned above. This simply means that direct neighbouring values are all higher (local minimum) or lower (local maximum). It is not necessary for values further away to be higher, or lower, respectively. Alternatively or additionally, it could be possible to relate the measured values to certain thresholds, either absolute and e.g. predetermined, or relative thresholds. In such a case, one could select only measured values below or above a certain value, or one could leave out certain points that exceed, or drop below, a certain (other) threshold, to avoid exceptional values due to inflammations or the like.

Various ways are possible to indicate the positions on the body part through the electrodes. One way could be to number the electrodes, and have the control means, or pattern recognition means, indicate the electrodes at (local) extreme values, by displaying their number(s) or the like. One could also apply the electrodes in a certain predetermined pattern to the body part, let the control means, or pattern recognition means, again indicate electrode positions of extreme values of the measured values. The pattern recognition means could already work with these data to display the pattern of extreme value positions on a (diagrammatical) display.

In a special embodiment, the plurality of said electrodes is provided in an electrode unit having a substantially fixed inter-electrode distance, and is preferably provided in a grid or matrix, in particular a tessellated pattern. In such an electrode unit, the electrodes can have a substantially fixed mutual distance in that for example relative rigid connections are provided between the electrodes. The inter-electrode distance need not be the same for each neighbouring pair of electrodes, as long as it is substantially fixed. If the positions of the electrodes are known, it is easy to indicate a pattern of local extreme values on a display. Note, however, that the inter-electrode distance between next-but-neighbours need not be substantially fixed, for the connections may be movable, such as pivotable around the electrode or the like. Furthermore, for this embodiment it suffices if the mutual distance is substantially fixed in use of the electrode unit, i.e. if the unit is applied to a body part. For example, if the electrode unit comprises a mesh or web or the like, in use it will be applied substantially fold-free, which also ensures substantially fixed distances. Note that in this embodiment, 'substantially fixed' would mean 'distance fixed within about 20% or better'. The distance would be measured across the surface, such as the surface of a leg.

There may be provided more than one electrode unit, in particular if a large part of a body, or more than one body part, is covered. In such a case, each such electrode unit may be embodied as disclosed herein.

Advantageously, the electrode unit is provided on or in a flexible body. Such a flexible body could comprise a substrate, for example a plastic pad. Rubber materials are excellent, because they have a high flexibility, which ensures that the flexible body is able to follow the contours of the body part, and a high electrical resistivity, which ensures reliable measurements. Furthermore, many rubbers are skin-friendly. Note that the inter-electrode distance could still be substantially fixed, depending on the flexibility.

In a particular embodiment, the electrode unit comprises a wearable device, in particular a garment for human wear. This enables the user to carry on with his normal duties. Examples depend on the body part to be treated, and could comprise a stocking or pants for a leg, and a jacket for the back or belly.

In particular, an average inter-electrode distance is at most 2 cm. In many cases, this ensures a sufficiently accuracy when measuring, without a too complex electrode unit. Preferably, the distance is at most 5 mm, which ensures a high accuracy in almost every case.

In an embodiment, the device according to the invention further comprises a memory device for storing at least one pattern. In this way, the device can be supplied with a pattern relating to a condition or body part to be treated. For example, such a pattern may be supplied on an information carrier, or could be a downloadable file, et cetera. Preferably, the memory device has at least one stored pattern, more preferably a plurality of stored patterns. In such a case, the device has been supplied e.g. in the factory with one or more patterns, that may be recognized by the pattern recognition means. Such patterns may have been previously determined on the body part to be treated. For example, a skilled person may have performed a single treatment with the device, in order to check its function and the collected measurements. The resulting pattern(s) may be stored for future use. In this way, very good reliability may be ensured, taking into account even small physiological variations. However, in practice, it turns out to be sufficiently reliable for one or more general patterns to be stored in the device.

In particular, the pattern, or at least one stored pattern comprises a surface pattern, preferably a surface pattern on said body part. This means that the pattern is represented as an unfolded pattern, in order to be able to couple the positions of the electrodes of the electrode unit to positions on a curved surface, such as the surface of most body parts.

In a particular embodiment, the pattern recognition means comprises a comparator arranged to compare a determined pattern with at least one stored pattern, and preferably to determine to which stored pattern the determined pattern corresponds. The comparator is a simple embodiment of a pattern recognition device, and could be embodied according to any other known pattern recognition technique. The comparator could e.g. be embodied as follows. First, it determines how many points are indicated in the stored pattern. If it is a pattern of five dots, the comparator could select the five measured spots with the highest, or lowest, measured value. Next, it could overlay its stored pattern, or one of them, over the five determined spots, in a first relative position. The comparator could then determine the sum of the distances between each measured spot and the (nearest) spot of the stored pattern. Then, by varying the relative position, this sum could be determined for another position, and so on. These other positions include rotations and even mirrored patterns. For a position where the sum is minimal, a further correction could be carried out by scaling the stored pattern, to correct for different dimensions. In such a way, the comparator, or the device as a whole, can determine where it has been positioned. It should be noted that the procedure described above is just one way to determine this position, and other patem recognition techniques known to the skilled person may also be provided and used.

In another embodiment, the device comprises at least one counterelectrode, preferably surrounding a surface with an area that is at least as large as a minimum surface area of the electrodes. The counterelectrode, which would be used to determine the electrical quantity, could be provided in various shapes and dimensions, some of which will be elucidated below. Note that it is also possible to use one or alternately various of the electrodes of the electrode unit. Note furthermore that in most cases, in particular when using one and the same counterelectrode for all measurements, the electrical quantity substantially only depends on the properties of the skin near, i.e. under, the electrode, since below the skin, most conduction is accounted for by blood, which is a very much better conductor than the higher skin layers. Hence the (relative) variation of e.g. resistance depends substantially only on the skin directly below the measuring electrode.

The counterelectrode could be provided as a serpentine, in between electrodes of the electrode unit, or as an electrode body that surrounds the electrodes, as an outer rim. There could also be provided a counterelectrode near every electrode, but, as mentioned above, the counterelectrode may be relatively far removed from the electrode without affecting measurement accuracy, while a too close counterelectrode could influence the measurement.

In a particular embodiment, the total surface area of the electrodes is at least 50%, preferably at least 85% of a surface area of the electrode unit measured within an outer limit touching the outermost electrodes of the plurality of electrodes. Herein, the surface area is to be calculated as the sum of the area between the outer rim of the electrode. This area could relate to the surface area of the electrode conductor proper, in the case of a solid, closed-surface electrode, or the surface area as surrounded by the outer rim of the electrode, such as in the case of ring-shaped conductors. It is advantageous to provide such a large total surface area, not only to increase the chance of measuring correct positions, i.e. not missing a local extreme value, but also because it is easier in this embodiment to measure with alow current density, which is less painful for the subject.

The device according to the invention advantageously further comprises a power source, preferably a battery. In such a case the device could function as a stand-alone, which increases mobility. It is however also possible to use mains or the like. In particular, the power source is provided in the electrode unit. This means that the measurements or the like may be carried, the electrode unit being powered by the power source.

Subsequently, the electrode unit could be coupled to the control unit and/or pattern recognition means for further processing of the data. This coupling may e.g. be brought about by means of a transmitter/receiver combination, or even an Internet or other network link. In this way, there is maximum freedom for the user. It is of course also, and with advantages, possible to provide the power source, the electrode unit and the control unit/pattern recognition means in one device.

In another embodiment, the device further comprises a warning means arranged to give off a warning signal if the pattern recognition means is unable to determine a pattern, in particular a pattern corresponding to a stored pattern. In this way, the user is warned that any of a number of faults/errors is present. For example, the electrode unit is incorrectly positioned, and either no pattern is recognizable, or an expected pattern is not found. Or, the device recognizes a pattern but this pattern is not the right pattern for treatment, and so on.

Preferably, the device comprises a display. In the display, not only may the pattern be indicated, but it may also be used to indicate further instructions for the user, such as "move the electrode unit downwards along the back" or "check skin contact" or the like.

In another embodiment, the device further comprises pattern selection means arranged to enable selection by a user of a stored pattern to which the determined pattern is to correspond. Thereto, the pattern selection means may comprise an input device, such as a switch, a knob or a keyboard. By being able to select a pattern actively, a user can actively determine what body part to treat. Note that in some cases, it could be required to contact a body part other than the one to be treated, due to nerve connections in the body. Especially in such cases, the present device is very useful, because then the appropriate spots on the body part should be found on a different body part. Prior art devices would at the most automatically look for extreme values on the body part to which the device is applied, which would give incorrect results.

Note that, in the present invention, selection of the body part to be treated could be carried out by directly entering the body part's name or other indication. The present device could then, by itself, select the appropriate body part, end or pattern.

In a preferred embodiment of the device according to the invention, the control unit comprises a treatment section arranged to provide treatment signals via at least one of the electrodes, preferably an electrode of a determined pattern. This means that the device is not only able to determine the treatment positions on the body part, but also to perform such a treatment. Thereto, a suitable electrical power source and/or signal generator could be provided, such as one that delivers pulses with a higher power or current or voltage than the signals for measuring. Such treatment signals may be selected from any known treatment. Furthermore, the treatment signals need not be supplied to the electrodes of the extreme measurement values, although this is often the case.

### BRIEF DESCRIPTION OF THE DRAWINGS

The invention will now be elucidated, with reference to the drawings that show non-limiting embodiments, and in which:
Figure 1 shows a diagram of an electro-therapeutic device according to the invention;
Figure 2 shows a human leg 20 to which an electrode unit 1 is applied;
Figure 3 shows the points 21 of minimum skin impedance that can be measured by the electrode unit 1 shown in Figure 2;
Figure 4A shows a detail of Figure 3;
Figure 4B shows the same detail of Figure 3 but in a different position;
Figure 5 shows an elbow of a person;
Figure 6 shows an underarm and a hand of a person;
Figure 7 shows an embodiment of a device according to the present invention;
   and
Figure 8 shows a flow diagram of a method according to the present invention.

### DETAILED DESCRIPTION OF EXAMPLES

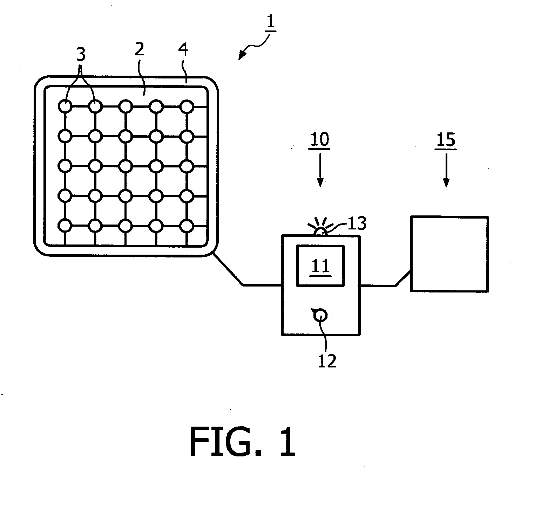

Figure 1 shows a diagram of an electro-therapeutic device according to the invention.

Herein, 1 denotes an electrode unit, 10 denotes a control unit and 15 denotes a memory device. The electrode unit 1 comprises a pad 2 and a matrix of electrodes 3, with a counterelectrode 4. The pad 2 comprises preferably an electrically insulating material such as most plastics. Advantageously, it is a skin-friendly material. An example is polyethylene. Shown here is a grid of 5 x 5 = 25 electrodes 3. Any other desired number is also possible. Furthermore, the grid is a square grid. If desired, any other pattern in which the mutual position of electrodes 3 is known is also possible. Note that the electrodes are not directly mutually electrically connected but each is connectable to the control unit 10.

The counterelectrode 4 could also be provided around the individual electrodes 3, or therebetween. In fact, the counterelectrode 4 is optional since each of the electrodes 3 could also serve as a counterelectrode for each of the other electrodes.

The electrodes 3 and the counterelectrode 4 can be applied to the skin in order to measure an electrical quantity such as impedance or resistance. The values of the electrical quantities that are measured by the electrodes 3 provide a list of position dependent values. These values may be communicated to the control device 10. This communication may be brought about by a direct electrical link, such as a cable, or for example by means of a transmitter - receiver combination. Similarly, the electrode unit 1 may comprise a power source such as a battery (not shown) or may be connected to the control unit that may comprise a power source.

The control unit 10 may comprise a display 11, a pattern selection switch 12 and an alarm signal device 13. The control unit 10 also comprises a measuring means (not shown separately) that is arranged to carry out measurements. For example, the measuring means comprises a voltage meter or an current meter. Preferably, a measurement output signal is output to the control unit.

The control unit for instance comprises a data processor, a memory, and a database that is stored in the memory and comprises several body part related configurations of nerve stimulation points. The user may choose from a list of body locations and/or programs. Alternatively, the control unit may determine a suitable program for a measured configuration of nerve stimulation points.

The display may for example be used to display individual measured values, or a pattern of extreme values, such as the lowest and/or highest values. The pattern may be evaluated, either by a user, a skilled person or by the control unit itself.

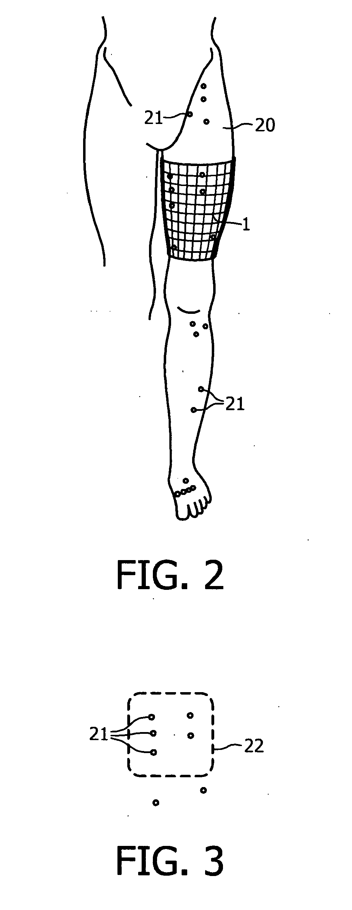

The display 11 could also indicate a desired treatment or a body part to be examined. By means of the pattern selection switch 12, a specific pattern to be looked for, or a body part or desired treatment could be selected. A desired pattern itself may already determine which body part is to be treated, or which treatment should be carried out. The selection of the pattern may also be determined by the device, in that a user enters what body part he wishes to treat, and/or what condition he wishes to treat, and the device selects the corresponding pattern from the stored patterns. If a pattern, body part or treatment is selected, the control unit 10 could compare a pattern that is measured by the electrode unit 1 with a pattern from the memory device 15. If the determined pattern corresponds to a stored pattern, the control unit 10 "knows" that the electrode unit 1 is correctly positioned on the body part, such that a treatment can be given. If the determined pattern corresponds to a stored pattern, the control unit 10 could treat the body part according to the desired treatment. This will be elucidated in the discussion of Figures 2-4. If the determined pattern does not corresponds to a stored pattern, for instance an alarm signal may be given that warns the user to shift the device or perform some other suitable action.

Figure 2 shows a human leg 20 to which an electrode unit 1 is applied. Also shown are electrode positions 21 representing for instance minimum skin impedance values.

The electrode unit 1 shown here comprises a kind of cuff or sleeve. In order to be wearable, the electrode unit 1 may comprise fastening means, such as elastic straps or the like. Note that the part of the electrode unit 1 should at the positions of the electrodes preferably not be elastic.

The electrode unit 1 can be placed onto the leg 20 by a user. The various electrodes, only indicated diagrammatically at the grid points of the electrode unit 1, may be used to determine local values of e.g. the skin impedance. The electrode(s) nearest the point 21 will show the lowest values of the skin impedance. Not all positions 21 of the complete leg 20 can be determined by this particular electrode unit 1. Only the positions 21 that are covered by the electrode unit 1 can be determined.

Figure 3 shows the points 21 of minimum skin impedance that can be measured by the electrode unit 1 shown in Figure 2. For simplicity, we will concentrate on the five points 21 within the dashed line 22. These five points have a particular constellation that for the moment is assumed to be unique for the body.

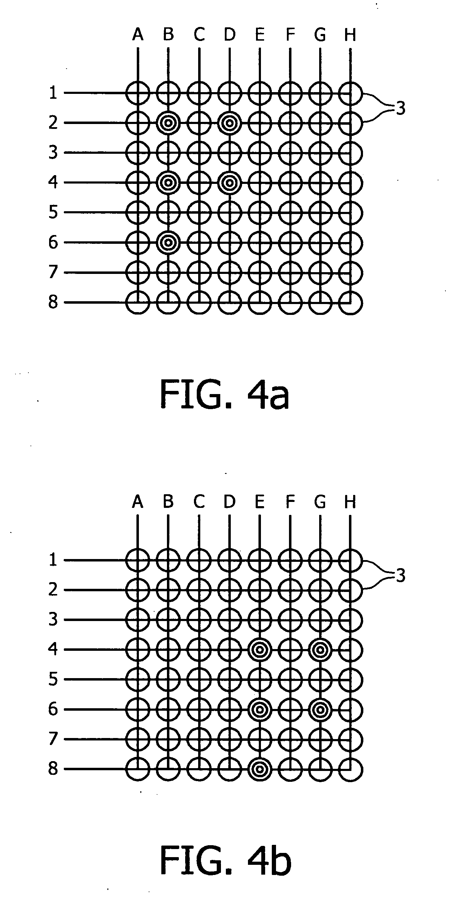

Figure 4A shows a grid of 8 x 8 electrodes 3 that correspond to the part within the dashed line in Figure 3. Open circles indicate electrodes that measure a relatively high skin impedance value, while black dots represent electrodes that measure a relatively low skin impedance value. The co-ordinates of electrodes may be denoted as for example 4B.

It can clearly be seen that the full pattern of Figure 3 within the dashed line 22 is present in Figure 4A. Hence the control unit (not shown here) will be able to determine the presence of this pattern by comparing it to the stored pattern for the upper leg. Furthermore, since the control unit knows that the (in this case 5) electrodes for this pattern are present at the locations 2B, 2D, 4B, 4D and 6B, the control unit may select a suitable treatment, that supplies a suitable voltage or the like to the indicated electrodes, or some other set of electrodes that would be proper for the treatment.

It is of course possible that the user applies the electrode unit 1 to the upper leg in a different position. One possibility is shown in Figure 4B, representing the same electrodes as shown in 4A, but in a different position.

Now, the electrodes 3 will show a different pattern, since this is shifted with respect to the electrodes. In the case of Figure 4B, the electrodes that measure the lowest impedance are electrodes 4E, 4G, 6E, 6G and 8E. Nevertheless, the pattern is still the same, and the control unit now determines that the pattern is still present but in a different position. The treatment can be adjusted accordingly.

In case the pattern of five low impedance positions is not measured by the electrode unit 1, the control unit will determine that the electrode unit 1 is not positioned correctly or does not make appropriate electrical contact to the skin. In this case, no treatment can be given. Alternatively, the measured pattern may be disrupted by for example a skin lesion or inflammation or the like. In any case, the alarm signal device 13 as shown in Figure 1 could generate an alarm signal warning the user that a treatment is not possible. Appropriate action should be taken and this may be indicated in the display 11 of the control unit.

Upon applying the electrode unit 1 to the patient, the electrode unit and the control unit 10 exchange a handshake. After the handshake procedure, the control unit 10 is aware of the position of the electrode unit on the body of the patient. For instance, if the electrode unit is still located on the upper leg, or that the electrode unit is now located on another body part, or on another patient.

Figure 2 shows an embodiment of the construction of a recognizing electrode unit 1. The control unit 10 can determine where the recognizing electrode unit 1 is positioned on the body of a patient using pattern recognition techniques on for instance the measured skin impedance. The control unit 10 must be able to drive and/or control all the electrodes 3 of the electrode unit. However, not all electrodes of the electrode unit 1 cover actual electrical nerve stimulation points 21, see for instance figure 2. To minimize the costs of the device according to the present invention, it is desirable to minimize the number of electrodes. At the same time, the device should preferably be suitable for most, for instance more than 95% or even 99%, of the patients.

It is therefore an object of a further embodiment of the present invention to maintain the ability to determine the location of the recognizing electrode unit on the body of a patient with a reduced number of electrodes.

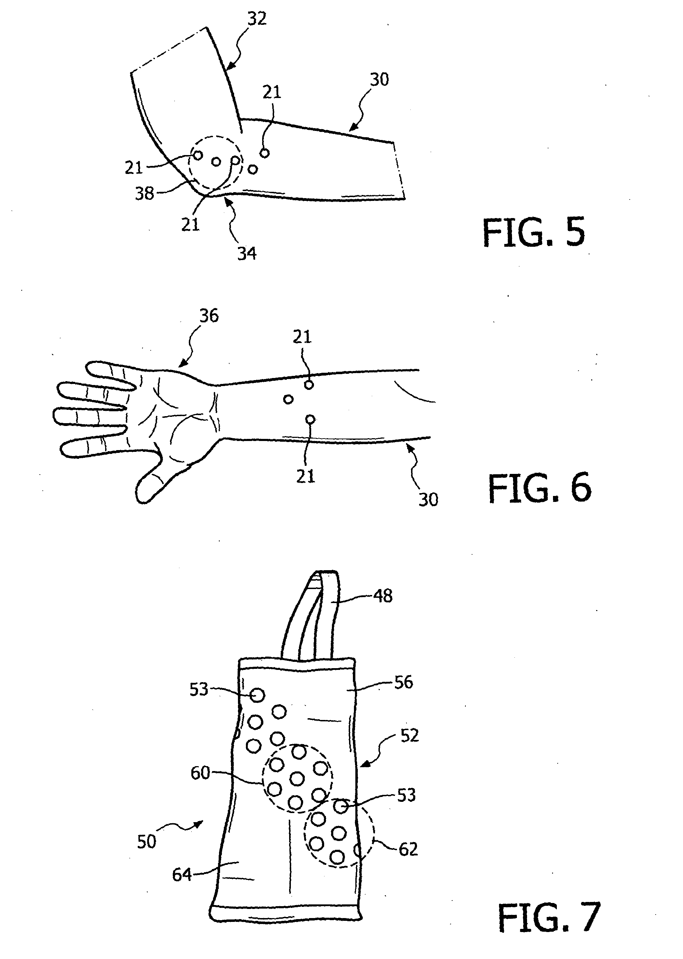

The actual location of the nerve stimulation points is slightly different from person to person. Figure 5 shows a forearm 30, upper arm 32 and elbow 34 of a patient.
Figure 6 shows the forearm 30 and a hand 36 of the patient. Figures 5 and 6 also show examples of manually determined nerve stimulation points 21. The circle 38 indicates the area where a skilled physiotherapist would expect to find potential nerve stimulation points, based on anatomical knowledge of the nervous system. The actual nerve stimulation points 21 are determined using impedance measurements.

A device 50 according to the invention includes an electrode pad 52. Figure 7 shows the inside of the electrode pad 52, which is provided with a reduced number of electrodes 53. I.e., the electrodes are provided on areas of the pad 25 that are most likely to cover the nerve stimulation points 21 of the patient, such as area 38 shown in Fig. 5. A control unit 10 (as shown in Fig. 1) may be electrically coupled to the pad 52 for driving the electrodes.

The pad 52 comprises an elastic cloth sleeve 56 for covering a body part of the patient, for instance an arm or leg. The electrodes may be metal parts that are embroidered in the cloth of the pad. A strap 58 is provided at an end of the sleeve 56. The strap 58 is intended for arranging the pad 52 in a predetermined position relative to the body part of the patient. If the body part is an arm, the strap will be positioned between the thump and the forefinger of the hand of the patient. If the body part is a leg, the strap will be positioned around the curvature of the underside of the foot. Other ways to position the electrode pad are also conceivable.

The pad 52 comprises a number of groups 60, 62, each group comprising a number of electrodes 53. The groups of electrodes are positioned at locations that are likely to cover nerve stimulation point 21 of the patient.

Techniques as described above may be used to detect movement of the pad 52 with respect to the body part of the patient, and/or to select the appropriate group of electrodes.

As shown in figure 7, a relatively large area 64 of the pad 52 lacks electrodes. Thus, an estimation of the locations of nerve stimulation points 21 is used to reduce the total number of electrodes on the pad 52. Reducing the number of electrodes reduces the number of corresponding electrical connections, reduces the complexity of the drive unit 10, and reduces overall manufacturing costs of the device.

The following options are available as an alternative for estimating the locations of nerve stimulation points beforehand.
1) To overcome inter-person variations of the nerve stimulation point locations, the area covered by the electrodes may be extended by increasing the number of groups of electrodes on the sleeve 52.
2) One could provide a range of sleeves 52, each having a different shape or size, et cetera. Upon purchase, the patient may try several sleeves. Subsequently, the patient can determine which sleeve has the optimum size or shape, or covers the most nerve stimulation points. As a further improvement, the sleeve could be fitted by a skilled professional.
3) In addition to option 2), a master device could be provided. The master device comprises a sleeve that is provided with a larger number of electrode groups. Using the master device, one may determine the actual positions of the nerve stimulation point of the patient, and which electrode groups are needed to measure or stimulate them. Using the thus obtained locations, a user-specific device can be obtained.

Above, the device of the present invention is described in relation to nerve stimulation points 21 that are related to locations of the skin having a local minimum or maximum of the electrical impedance. Other criteria than skin impedance may however be preferred. There might be several reasons why other selection criteria are preferred. The appropriate nerve stimulation points and their corresponding electrodes may for instance be selected as follows:
1) Sequentially, all electrodes of the device are provided with an electrical signal during a predetermined time interval. The time interval may for instance be in the order of several seconds. Upon sequential stimulation of the electrodes, the patient will provide feedback. Using the feedback, the electrodes for obtaining the desired effect or result can be selected.
2) First, the skin impedance is measured. The electrode that is located at the position where the skin impedance is the lowest is than activated, and the patient provides a score of the effect of the activated electrode. The score could for instance be relative to the first effect, or relative to a predetermined score or effect. Subsequently, the electrode that is located at the position of the second lowest skin impedance is activated, and scored by the patient. Scoring of the effect of activating the electrodes continues until the patient has found the optimal locations for, for instance, pain relief.
3) In the case of using the device 50 shown in Fig. 7, multiple groups of electrodes are stimulated.
4) In the case of using the device 50 shown in Fig. 7, one group of electrodes is stimulated using a first pulse pattern, and a second group of electrodes is stimulated using another, second pulse pattern, et cetera.

The above described selection criteria concern feedback that is provided by the patient. The electrodes that will be used for stimulation are selected, based on said feedback. Using the feed-back of the patient to select electrodes provides the patient with a certain level of control over the treatment. Also, the electrodes may be selected using feedback of the patient if automatic selection of electrodes based on skin impedance measurements fails, or turns out to be sub-optimal.

In an embodiment, a quantitative sensory test (QST) is used to monitor and/or evaluate the above described treatment. Besides, the patient may be informed regarding the progress of the treatment, to motivate the patient to continue the treatment.

The quantitative sensory test in general used to evaluate the function of nerve fibers. The large nerve fibers carry sensations of vibrations; the medium nerve fibers carry sensations of cold; and the small nerve fibers carry sensations of heat. QST is especially effective because it provides information about even the smallest nerve fibers. QST is used to diagnose conditions that involve sensory abnormalities such as pain, burning, tingling, and numbness in the arms, legs, or trunk of the body.

During the QST procedure, the healthcare professional will administer for instance various hot, cold, and vibrating stimulations to both the affected and non-affected areas of the body of the patient. The stimulation is brief, and should be no more than mildly uncomfortable when testing a response to heat and cold. The patient will be asked to tell the healthcare professional when he feels the stimulation and what type of sensation he feels.

The control unit 10 (Fig. 1) may exchange data with a user. The control unit may comprise a base station (not shown) that is connected to a data processor or computer. Upon placing the (portable) control unit in or on the base station, the control unit may exchange any available data with the base station. A healthcare professional may thus check certain parameters, such as defects, settings, or faithfulness to the therapy. The parameters may be changed or corrected via the base station.

Using the base station, the device of the present invention may for instance be integrated in Philips interactive healthcare systems or remote patient management technology, such as Motiva™ (see for instance Press Release, "Frost & Sullivan presents Philips with 2005 Technology Leadership Award", London, UK - 12 December 2005).

In an embodiment, the control unit includes a slot for inserting a memory card or smart card having a memory and one or more programs. The smart card enables the control unit to deal with a specific disorder, or to treat a specific part of the body, using a suitable program. Every program is for instance adapted for stimulating a predetermined number of the electrodes in a predetermined fashion. The healthcare professional may provide the patient with the smart card. Otherwise, programs may be available via the internet or at a shop. The programs may be self-learning. Self-learning indicates for instance adapting to the settings that are most frequently used during a certain time frame, for instance the last week or month.

Besides programs for treating disorders, electrode pads and/or programs that are specifically adapted for massaging parts of the body may be available.

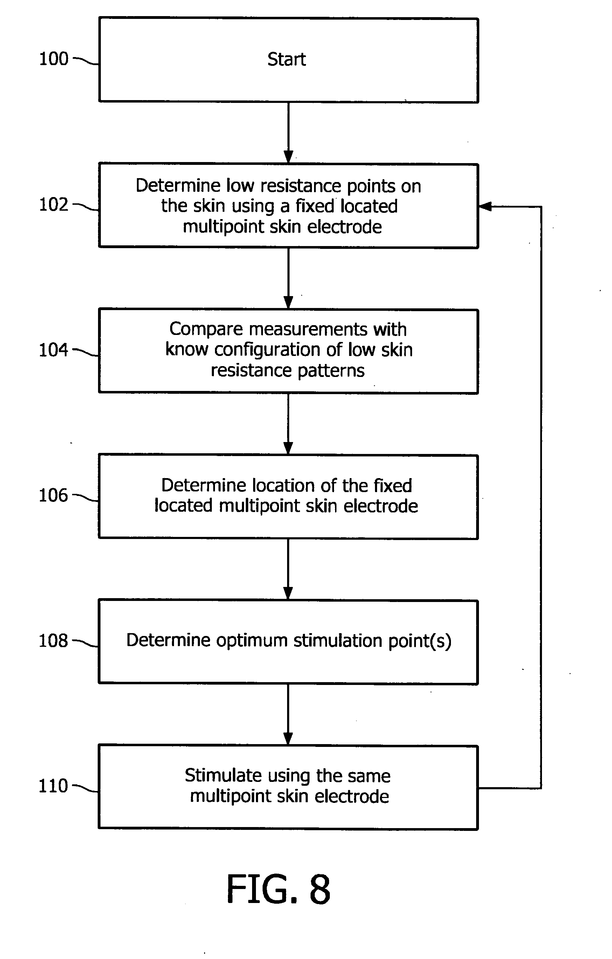

A method of using the device according to the present invention is described below in relation to Fig. 8.

The method starts at step 100. Previously, a therapist applying transcutaneous electrical nerve stimulation (TENS) would search manually for the optimal stimulation points. The device of the present invention however is able to determine, for instance, low electrical skin resistance points on a certain body part of the patient automatically (step 102).

At step 104, the device compares the configuration of measured low resistance points with configurations of low skin resistance points that are stored in a database of the control unit. The device uses pattern recognition to determine a matching pattern in the database.

At step 106, the matching pattern is used to determine to location of the electrode pad at the body of the patient.

At step 108, the device determines the optimal nerve stimulation point(s) for the selected treatment.

At step 110, the device stimulates the optimal nerve stimulation point(s) for the selected treatment using the electrode pad.

The present invention not only simplifies the search procedure for the optimal location of nerve stimulation points, but also potentially obviates the need for a therapist completely, because anatomical knowledge of stimulations point is no longer required.

The device of the present invention measures skin resistance as a parameter for localization of the stimulation points, whereas the same device of used for actual treatment. The nerve stimulation points are found in configurations on the skin that are specific for various body areas or parts. The device of the present invention compares the found patterns to patterns that are stored in a database. Recognized patterns are used to select a proper program for treating the body part with the electrode pad. The present invention enables consumers to apply and re-apply an electrode for TENS treatment without the intervention of a therapist.

The device of the present invention is able to recognize one or more patterns of nerve stimulation points, to determine the position of the matrix of electrodes, and/or to determine the optimal stimulation point(s) for the envisioned treatment.

The present invention is not limited to the above described embodiments. Many variations and combinations of features of the embodiments are possible, within the scope of the appended claims.

## Claims

1. Electro-therapeutic device for application on a human or animal body part (20), comprising
- a plurality of electrodes (3);
- measuring means operably electrically connected to the plurality of electrodes (3) and arranged to measure a value of an electrical quantity for each electrode in at least a subset of the plurality of electrodes (3); and
- a control unit (10) coupled to the measuring means and arranged to process the measured values,
**characterized in that** the control unit (10) further comprises pattern recognition means arranged to determine an electrode position on the human or animal body using pattern recognition on a pattern in the measured values.

2. The device according to claim 1, wherein the electrical quantity comprises skin impedance, skin resistance or a relative skin sensory perception.

3. The device according to claim 1 or 2, wherein the determined pattern comprises a subgroup of the plurality of electrodes (3) that indicate a local extreme value of said electrical quantity, preferably positions of the electrodes (3) of the subgroup on said body part (20).

4. The device according to any preceding claim, wherein said plurality of electrodes (3) is provided in an electrode unit (1) having a substantially fixed inter-electrode distance, and is preferably provided in a grid or matrix, in particular a tessellated pattern.

5. The device according to claim 4, wherein the electrode unit (1) is provided on or in a flexible body (2).

6. The device according to claim 4 or 5, wherein the electrode unit (1) comprises a wearable device, in particular a garment for human wear.

7. The device according to any of claims 4-6, wherein an average inter-electrode distance is at most 2 cm, preferably at most 5 mm.

8. The device according to any preceding claim, further comprising a memory device (15) for storing at least one pattern, preferably with at least one stored pattern, more preferably with a plurality of stored patterns.

9. The device according to claim 8, wherein the pattern recognition means comprise a comparator arranged to compare a determined pattern (21) with at least one stored pattern, and preferably to determine to which stored pattern the determined pattern (21) corresponds.

10. The device according to any preceding claim, wherein the determined pattern (21) and/or at least one stored pattern comprises a surface pattern, preferably a surface pattern on said body part.

11. The device according to any preceding claim, comprising at least one counterelectrode (4), preferably surrounding a surface with an area that is at least as large as a minimum surface area of the electrodes (3).

12. The device according to any of claims 4-11, wherein the total surface area of the electrodes (3) is at least 50%, preferably at least 85% of a surface area of the electrode unit (1) measured within an outer limit touching the outermost electrodes of the plurality of electrodes (3).

13. The device according to any preceding claim, further comprising a power source, preferably a battery, in particular a power source being provided in the electrode unit (1).

14. The device according to any preceding claim, further comprising a warning means (13) arranged to generate a warning signal if the pattern recognition means are unable to determine a pattern, in particular a pattern corresponding to a stored pattern.

15. The device according to any preceding claim, further comprising a pattern selection means (12) arranged to enable selection by a user of a stored pattern to which the determined pattern (21) is to correspond.

16. The device according to any preceding claim, wherein the control unit (10) comprises a treatment section arranged to provide treatment signals via at least one of the electrodes (3), preferably an electrode of a determined pattern (21).

## Patentansprüche

1. Elektrotherapeutisches Gerät zur Anwendung auf einem Teil (20) eines Körpers eines Menschen oder Tieres, mit:
- einer Vielzahl von Elektroden (3);
- Messmitteln, die mit der Vielzahl von Elektroden (3) betriebsfähig elektrisch verbunden und so eingerichtet sind, dass sie einen Wert einer elektrischen Größe für jede Elektrode in zumindest einer Teilmenge der Vielzahl von Elektroden (3) messen; sowie
- einer Steuereinheit (10), die mit den Messmitteln gekoppelt und so eingerichtet ist, dass sie die gemessenen Werte verarbeitet,
**dadurch gekennzeichnet, dass** die Steuereinheit (10) weiterhin Mustererkennungsmittel umfasst, die so eingerichtet sind, dass sie durch Mustererkennung auf einem Muster in den gemessenen Werten eine Elektrodenposition auf dem Körper eines Menschen oder Tieres ermitteln.

2. Gerät nach Anspruch 1, wobei die elektrische Größe Hautimpedanz, Hautwiderstand oder eine relative hautsensorische Wahrnehmung umfasst.

3. Gerät nach Anspruch 1 oder 2, wobei das ermittelte Muster eine Teilgruppe der Vielzahl von Elektroden (3) umfasst, die einen lokalen Extremwert der elektrischen Größe, vorzugsweise Positionen der Elektroden (3) der Teilgruppe auf dem Körperteil (20), angeben.

4. Gerät nach einem der vorangegangenen Ansprüche, wobei die Vielzahl von Elektroden (3) in einer Elektrodeneinheit (1) mit einem im Wesentlichen festen Abstand zwischen den Elektroden und vorzugsweise in einer Gitter- oder Matrixanordnung, insbesondere einem Mosaikmuster, vorgesehen ist.

5. Gerät nach Anspruch 4, wobei die Elektrodeneinheit (1) auf oder in einem flexiblen Körper (2) vorgesehen ist.

6. Gerät nach Anspruch 4 oder 5, wobei die Elektrodeneinheit (1) aus einem tragbaren Gerät, insbesondere einem von Menschen getragenen Kleidungsstück, besteht.

7. Gerät nach einem der Ansprüche 4-6, wobei ein durchschnittlicher Abstand zwischen den Elektroden höchstens 2 cm, vorzugsweise höchstens 5 mm, beträgt.

8. Gerät nach einem der vorangegangenen Ansprüche, welches weiterhin eine Speichereinrichtung (15) zum Speichern von mindestens einem Muster, vorzugsweise mit mindestens einem gespeicherten Muster, noch besser mit einer Vielzahl von gespeicherten Mustern, umfasst.

9. Gerät nach Anspruch 8, wobei die Mustererkennungsmittel einen Komparator umfassen, der so eingerichtet ist, dass er ein ermitteltes Muster (21) mit mindestens einem gespeicherten Muster vergleicht und vorzugsweise ermittelt, welchem gespeicherten Muster das ermittelte Muster (21) entspricht.

10. Gerät nach einem der vorangegangenen Ansprüche, wobei das ermittelte Muster (21) und/oder das mindestens eine gespeicherte Muster ein Oberflächenmuster, vorzugsweise ein Oberflächenmuster auf dem Körperteil, umfasst.

11. Gerät nach einem der vorangegangenen Ansprüche, das mindestens eine Gegenelektrode (4) umfasst, die vorzugsweise eine Oberfläche mit einer Fläche, die mindestens so groß wie eine Mindestoberfläche der Elektroden (3) ist, umgibt.

12. Gerät nach einem der Ansprüche 4-11, wobei die Gesamtoberfläche der Elektroden (3) mindestens 50%, vorzugsweise mindestens 85%, einer Oberfläche der Elektrodeneinheit (1), gemessen innerhalb einer die äußersten Elektroden der Vielzahl von Elektroden (3) berührenden, äußeren Grenze, ausmacht.

13. Gerät nach einem der vorangegangenen Ansprüche, welches weiterhin eine Energiequelle, vorzugsweise eine Batterie, insbesondere eine in der Elektrodeneinheit (1) vorgesehene Energiequelle, umfasst.

14. Gerät nach einem der vorangegangenen Ansprüche, welches weiterhin ein Warnmittel (13) umfasst, das so ausgebildet ist, dass es ein Warnsignal erzeugt, wenn die Mustererkennungsmittel nicht imstande sind, ein Muster, insbesondere ein Muster entsprechend einem gespeicherten Muster, zu ermitteln.

15. Gerät nach einem der vorangegangenen Ansprüche, welches weiterhin ein Musterauswahlmittel (12) umfasst, welches so ausgebildet ist, dass es die von einem Anwender getroffene Auswahl eines gespeicherten Musters, dem das ermittelte Muster (21) entsprechen soll, ermöglicht.

16. Gerät nach einem der vorangegangenen Ansprüche, wobei die Steuereinheit (10) einen Behandlungsabschnitt umfasst, der so ausgebildet ist, dass er Behandlungssignale über mindestens eine der Elektroden (3), vorzugsweise eine Elektrode eines zuvor festgelegten Musters (21), vorsieht.

## Revendications

1. Dispositif électro-thérapeutique pour application sur une partie corporelle d'un humain ou animal (20), comprenant
- une pluralité d'électrodes (3) ;
- des moyens de mesure électriquement connectés de manière opérationnelle à la pluralité d'électrodes (3) et agencés pour mesurer une valeur d'une quantité électrique pour chaque électrode dans au moins un sous-ensemble de la pluralité d'électrodes (3) ; et
- une unité de commande (10) couplée aux moyens de mesure et agencée pour traiter les valeurs mesurées,
**caractérisé en ce que** l'unité de commande (10) comprend en outre des moyens de reconnaissance de motif agencés pour déterminer une position d'électrode sur le corps humain ou animal au moyen d'une reconnaissance de motif sur un motif dans les valeurs mesurées.

2. Dispositif selon la revendication 1, dans lequel la quantité électrique comprend l'impédance de la peau, la résistance de la peau ou une perception sensorielle relative de la peau.

3. Dispositif selon la revendication 1 ou 2, dans lequel le motif déterminé comprend un sous-groupe de la pluralité d'électrodes (3) qui indique une valeur extrême locale de ladite quantité électrique, de préférence des positions des électrodes (3) du sous-groupe sur ladite partie corporelle (20).

4. Dispositif selon l'une quelconque des revendications précédentes, dans lequel ladite pluralité d'électrodes (3) est disposée dans une unité d'électrode (1) ayant une distance inter-électrode sensiblement fixe et est de préférence disposée dans une grille ou une matrice, en particulier un motif en mosaïque.

5. Dispositif selon la revendication 4, dans lequel l'unité d'électrode (1) est disposée sur ou dans un corps flexible (2).

6. Dispositif selon la revendication 4 ou 5, dans lequel l'unité d'électrode (1) comprend un dispositif portable, en particulier un vêtement à porter par un humain.

7. Dispositif selon l'une quelconque des revendications 4 à 6, dans lequel une distance moyenne inter-électrode est d'au plus 2 cm, de préférence d'au plus 5 mm.

8. Dispositif selon l'une quelconque des revendications précédentes, comprenant en outre un dispositif de mémoire (15) pour stocker au moins un motif, de préférence avec au moins un motif stocké, de manière davantage préférée avec une pluralité de motifs stockés.

9. Dispositif selon la revendication 8, dans lequel les moyens de reconnaissance de motif comprennent un comparateur agencé pour comparer un motif déterminé (21) avec au moins motif stocké, et de préférence pour déterminer à quel motif stocké correspond le motif déterminé (21).

10. Dispositif selon l'une quelconque des revendications précédentes, dans lequel le motif déterminé (21) et/ou au moins un motif stocké comprend un motif de surface, de préférence un motif de surface sur ladite partie corporelle.

11. Dispositif selon l'une quelconque des revendications précédentes, comprenant au moins une contre-électrode (4), de préférence entourant une surface avec une superficie qui est au moins aussi grande qu'une superficie minimale des électrodes (3).

12. Dispositif selon l'une quelconque des revendications 4 à 11, dans lequel la superficie totale des électrodes (3) est d'au moins 50 %, de préférence au moins 85 % d'une superficie de l'unité d'électrode (1) mesurée dans une limite externe touchant les électrodes les plus externes de la pluralité d'électrodes (3).

13. Dispositif selon l'une quelconque des revendications précédentes, comprenant en outre une source d'énergie, de préférence une batterie, en particulier une source d'énergie disposée dans l'unité d'électrode (1).

14. Dispositif selon l'une quelconque des revendications précédentes, comprenant en outre un moyen d'avertissement (13) agencé pour générer un signal d'avertissement si les moyens de reconnaissance de motif sont incapables de déterminer un motif, en particulier un motif correspondant à un motif stocké.

15. Dispositif selon l'une quelconque des revendications précédentes, comprenant en outre un moyen de sélection de motif (12) agencé pour permettre la sélection par un utilisateur d'un motif stocké auquel le motif déterminé (21) doit correspondre.

16. Dispositif selon l'une quelconque des revendications précédentes, dans lequel l'unité de commande (10) comprend une section de traitement agencée pour fournir des signaux de traitement via au moins l'une des électrodes (3), de préférence une électrode d'un motif déterminé (21).
